# EUROPÄISCHE PATENTSCHRIFT

(11) **EP 1 096 951 B1**
(45) Veröffentlichungstag und Bekanntmachung des Hinweises auf die Patenterteilung: **14.08.2002**
(21) Anmeldenummer: 98942559.0
(22) Anmeldetag: 15.07.1998
(51) Int. Cl.: A61K 38/48, A61P 7/00

(54) **VERWENDUNG VON BROMELAINPROTEASEN ZUR HEMMUNG DER BLUTGERINNUNG**
USE OF BROMELAIN PROTEASES FOR INHIBITING BLOOD COAGULATION
UTILISATION DE BROMELAINE PROTEASES POUR EMPECHER LA COAGULATION DU SANG

(43) Veröffentlichungstag der Anmeldung: 09.05.2001
(73) Patentinhaber: URSAPHARM Arzneimittel GmbH & Co. KG, D-66129 Saarbrücken (DE)
(72) Erfinder: MAURER, Rainer, D-14129 Berlin (DE); ECKERT, Klaus, D-13125 Berlin (DE); GRABOWSKA, Edyta, D-10318 Berlin (DE); ESCHMANN, Klaus, D-66271 Kleinblittersdorf (DE)
(74) Vertreter: Becker Kurig Straus
(86) Internationale Anmeldenummer: EP9804406
(87) Internationale Veröffentlichungsnummer: WO0003729

(56) Entgegenhaltungen:
- WO-A-98/38291
- TAUSSIG S J ET AL: "Bromelain, the enzyme complex of pineapple (ananas comosus) and its clinical application. An update" JOURNAL OF ETHNOPHARMACOLOGY, Bd. 22, Nr. 2, 1988, Seite 191-203 XP002097864
- HARRACH T ET AL: "ISOLATION AND PARTIAL CHARACTERIZATION OF BASIC PROTEINASES FROM STEM BROMELAIN" JOURNAL OF PROTEIN CHEMISTRY, Bd. 14, Nr. 1, Januar 1995, Seiten 41-52, XP002069063 in der Anmeldung erwähnt
- SUNNY M C ET AL: "Effect of fabrication, sterilization and mediators-blood compatibility of polyurethanes" JOURNAL OF BIOMATERIALS APPLICATIONS, Bd. 6, Nr. 3, Januar 1992, Seite 261-273 XP002097865
- DATABASE WPI Derwent Publications Ltd., London, GB; AN 89-019644 XP002097957 & JP 63 295515 A (KAO CORP), Januar 1988

## Beschreibung

Diese Erfindung betrifft die Verwendung bestimmter basischen Bromelainproteasen zur Herstellung eines Medikamentes zur Hemmung des Blutgerinnungssystems, insbesondere zur Stimulation der Plasmin-Bildung, der Hemmung der Fibrin-Bildung und der Hemmung der Adhäsion humaner Thrombozyten an Endothelzellen.

Bromelain ist ein aus Pflanzen der Familie Bromeliaceae isolierbares Gemisch unterschiedlichster Proteine, dessen genaue Zusammensetzung aufgrund der Komplexität und Vielfalt der darin enthaltenen Komponenten noch nicht vollständig charakterisiert werden konnte. Es konnte jedoch gezeigt werden, daß Bromelain verschiedene Phosphatasen, Cellulasen, Glycosidasen, Cystein-Proteasen und deren Peptid-Inhibitoren, sowie weitere, noch nicht näher identifizierte Komponenten enthält. Die stoffliche und mengenmäßige Zusammensetzung von Bromelain variiert jedoch in Abhängigkeit von der Herkunft und dem Isolierungsverfahren aus der jeweiligen Quelle, so daß unterschiedliche Verfahren zur Isolierung des Rohprodukts, dessen Standardisierung sowie zur Aufreinigung bestimmter, darin enthaltener Komponenten entwickelt wurden.

Einige der Komponenten von Bromelain konnten bereits näher identifiziert werden. So wird von Murachi et al., in The Journal of Biological Chemistry **1** (1960), 99-107 berichtet, daß Bromelain mindestens 5 ähnlich wirkende Proteasen mit unterschiedlicher Substratspezifität und unterschiedlichem pH-Optimum enthält.

Bei mit Bromelain durchgeführten Studien wurde weiter gefunden, daß sich dieses Gemisch auch als Arzneimittel zur Behandlung verschiedener Erkrankungszustände einsetzen läßt.

So wird in der DE 41 30 221 die Verwendung von Papain und/oder Trypsin, von dem Bromelaingemisch abgeleitete, bestimmte proteolytische Enzyme, zur Herstellung eines Arzneimittels vorgeschlagen, das zur Behandlung von Autoimmunerkrankungen geeignet sein soll. Gemäß dieser Patentschrift soll das Papain bzw. das Trypsin auf bei der Entstehung von Autoimmunerkrankungen beteiligte Proteine einwirken, die eine C_{H}2-Domäne aufweisen.

In der DE 43 02 060 wird weiter die Verwendung von Bromelain als Gemisch zur Krebstherapie und/oder Metastasen-Prophylaxe gelehrt. Dabei wird davon ausgegangen, daß Bromelain auf CD44, ein auf unterschiedlichen Zellen des Organismus vorkommendes, stark glycosyliertes Oberflächenprotein einwirkt, dem eine Rolle bei der Tumorentwicklung nachgesagt wird.

In der WO 95/00169 wird die Isolierung und Charakterisierung einer Protease aus dem Bromelaingemisch erläutert, die auf den Syntheseweg cyclischer Nucleotide einwirkt. Das als "Stem Bromelain Protease" bezeichnete Enzym weist 213 Aminosäuren auf und soll Erkrankungen, wie der Tumorentstehung, Atherosclerose oder auch bakteriellen Infektionen vorbeugen können.

In der JP-A-63295515 wird ein anti-koagulativ wirkendes, thrombolytisches Arzneimittel beschrieben, in welchem eine Protease als aktiver Bestandteil enthalten ist. Die Protease dieses Arzneimittels wirkt als Plasminogen-Aktivator.

Bezüglich des weiteren Stands der Technik wird von Taussig et al. im Journal of Ethnopharmacology **22** (1988), 191-203 die pharmzeutische Verwendbarkeit des Komponentenkomplexes Bromelain dargestellt. Diesem Wirkstoffkomplex werden neben der Wachstumshemmung von malignen Zellen und der Hemmung der Thrombozytenaggregation auch fibrinolytische Aktivtät, anti-inflammatorische Wirkung und wundreinigende Eigenschaften der Haut zugeschrieben. Der Komponentenkomplex Bromelain zeigt biologische Aktivität bezüglich der Modifizierung des Krebswachstums, der Blutgerinnung oder infammatorischer Veränderungen sowie eine Verbesserung der Arzneimittelaufnahme.

Aufgrund der Entwicklung auf dem Gebiet der Aufreinigungstechniken konnten weitere Komponenten aus dem Bromelaingemisch isoliert und teilweise auch charakterisiert werden. So wurde von Eckert et al., in The Journal of Protein Chemistry **14** (1995), 41-52 offenbart, daß Bromelain mindestens 8 basische Proteasen enthält, die über FPLC-Kation-Austauschchromatographie fraktionert werden konnten. Auch zwei Formen säurer Proteasen konnten nachgewiesen werden (Maurer et al., Journal of Protein Chemistry **17** (1998), 351-361.

Obwohl für Bromelain bereits verschiedenste medizinische Anwendungsgebiete erkannt wurden, besteht ein Bedarf, weitere Anwendungen für Bromelain aufzufinden. Dabei wäre es aufgrund des nicht vollständig verstandenen Zusammenwirkens der einzelnen Komponenten im Gemisch wünschenswert, für das jeweilige Einsatzgebiet nicht das Gemisch selbst einzusetzen, sondern nur die für den jeweiligen Zweck verantwortliche Komponente des Gemisches. Dabei tritt jedoch ein Problem auf, da nicht vorhergesagt werden kann, ob einzelne Komponenten im isolierten Zustand ohne andere, im Bromelaingemisch noch vorhandene zusätzliche Stoffe selbst wirksam sind, oder ob diese vielmehr zusätzliche, im Bromelaingemisch vorhandene, jedoch noch nicht erkannte Komponenten als Hilfsstoffe benötigen.

Eine Aufgabe der Erfindung besteht daher darin, weitere Verwendungsmöglichkeiten für Bromelain, insbesondere deren Komponenten aufzuzeigen.

Eine weitere Aufgabe der vorliegenden Erfindung besteht darin, die jeweils für den medizinischen Zweck verantwortliche Komponente(n) aufzufinden und einer medizinischen Verwendung zugänglich zu machen.

Die Erfinder haben umfangreiche Studien durchgeführt und überraschenderweise gefunden, daß allein mit bestimmten im Bromelaingemisch vorhandenen basischen Bromelainproteasen, ohne weitere, in diesem Gemisch vorhandene Komponenten eine Hemmung der Blutgerinnung erzielt werden kann.

Die erfindungsgemäß verwendeten basischen Bromelainproteasen sind die, die gemäß dem von Eckert et al., in The Journal of Protein Chemistry **14** (1995), 41 - 52 beschriebenen Verfahren als Fraktionen F4, F5 F9 gewonnenen werden.

Die vorstehend aufgeführte Aufgabe wird daher dadurch gelöst, indem die oben genannten im Bromelaingemisch vorhandenen basischen Bromelainproteasen zur Herstellung eines Medikamentes zur Hemmung der Blutgerinnung verwendet werden.

Es hat sich gezeigt, daß durch diese basischen Bromelainproteasen insbesondere die Bildung von Plasmin gefördert, während die Bildung von Fibrin sowie die Adhäsion von Thrombozyten an Endothelzellen, Vorgänge, die bei der Blutgerinnung eine wichtige Rolle spielen, gehemmt wird.

Die in der Fraktion F4 enthaltene Protease weist ein Molekulargewicht von etwa 24,4 KDa auf und besitzt ein Optimum der Aktivität bei einem pH-Wert im Bereich von ca. 4 bis 5,5. Die Protease umfaßt weiter die folgende Aminosäuresequenz:

Val Pro Gln Ser Ile Asp Trp Arg Asp Tyr
Gly Ala Val Thr Ser Val Lys Asn Gln Asn

Die in der Fraktion F5 enthaltene Protease weist ein Molekulargewicht von etwa 24,5 KDa auf und besitzt ein Optimum der Aktivität bei einem pH-Wert im Bereich von ca. 3,5 bis 5. Die. Protease umfaßt weiter die folgende Aminosäuresequenz:

Val Pro Gln Ser Ile Asp Trp Arg Asp Tyr
Gly Ala Val Thr Ser Val Lys Asn Gln Asn

Die in der Fraktion F9 enthaltene Protease weist ein Molekulargewicht von etwa 23,4-KDa auf und besitzt ein Optimum der Aktivität bei einem pH-Wert im Bereich von ca. 6 bis 8. Die Protease umfaßt weiter die folgende Aminosäuresequenz:

Val Pro Gln Ser Ile Asp Trp Arg Asp Ser
Gly Ala Val Thr Ser Val Lys Asn Gln Gly

Es hat sich nun überraschenderweise gezeigt, daß eine wirksame Hemmung der Blutgerinnung unter Verwendung der genannten basischen Bromelainproteasen erreicht werden kann, und daß diese Hemmung lediglich mit diesen aus dem Bromelaingemisch isolierten basischen Bromelainproteasen erreichbar ist, ohne daß dabei andere, im Bromelaingemisch vorhandene, zusätzliche Komponenten eine Rolle spielen würden.

Die erfindungsgemäß zu Verwendenden basischen Bromelainproteasen können einem Subjekt in einer bereits für das Bromelaingemisch bekannten Art und Weise verabreicht werden, d.h. durch intravenöse oder intraperitoneale Gabe oder vorzugsweise oral, wobei die Wirkstoffe dann mit im Stand der Technik gebräuchlichen Exzipienten formuliert werden, um die Proteasen in aktiver Form durch den Magen-Darm-Trakt zu bringen und eine systemische Verfügbarkeit zu gewährleisten.

Diese basischen Bromelainproteasen können nach herkömmlichen Verfahren isoliert werden. Insbesondere eine Aufreinigung, wie von Eckert et al., in The Journal of Protein Chemistry **14** (1995), 41 - 52 und von Maurer et al., in The Journal of Protein Chemistry **17** (1998) angegeben, kann dazu eingesetzt werden. Nach Aufreinigung können die Proteasen ansequenziert werden und das entsprechende Gen mittels molekularbiologischer Methoden aus dem Genom von beispielsweise der Ananas isoliert werden. Mittels molekularbiologischer Methoden kann dann in herkömmlicher Art und Weise ein rekombinantes Protein bereitgestellt werden.

Die Erfindung wird nun anhand der folgenden Beispiele näher beschrieben, die lediglich zur Erläuterung dienen und nicht als Beschränkung der vorliegenden Erfindung gedacht sein sollen.

Die in der vorliegenden Erfindung zum Einsatz kommenden basischen Bromelainproteasen werden isoliert wie von Eckert et al., in The Journal of Protein Chemistry **14** (1995), 41 - 52 sowie von Maurer et al., in The Journal of Protein Chemistry **17** (1998) angegeben. Der Inhalt dieser Druckschriften wird hiermit vollumfänglich in den Offenbarungsgehalt der vorliegenden Anmeldung miteinbezogen.

Als Beispiel der Auswirkungen der genannten basischen Bromelainproteasen auf die Blutgerinnung wird stellvertretend die gemäß vorstehenden Dokumenten isolierte Fraktion F9 herangezogen.

### Wirkungen von Bromelain F9 auf die Fibrinolyse

Zum Nachweis der Wirkung wird eine Bestimmungsmethode eingesetzt, die auf der Verwendung eines chromogenen Substrates in einem photometrischen Nachweissystem basiert. Mit dem verwendeten Testkit Berichrom-Plasminogen (Fa. Behring) wird das Plasminogen der Probe durch Streptokinase in einen Komplex überführt. In dem kinetischen Test kann die Freisetzung von Plasmin durch Zugabe des Plasmin-Substrates über die Extinktionszunahme quantitativ erfaßt werden.

### Beispiel 1

In diesem Experiment wird die fibrinolytische Aktivität von Bromelain F9 der von Bromelain Base Powder (Rohprodukt) und Streptokinase gegenübergestellt.

Ausgangsmaterial für die Bestimmung der fibrinolytischen Aktivität der zu testenden Protease Bromelain F9 ist das Citratplasma gesunder Spender. 9 Teile Venenblut werden mit 1 Teil Natriumcitratlösung (0,11 Mol/l) gemischt und anschließend 10 min (1500 x g) zentrifugiert. Streptokinase, Urokinase, tPA, Plasmin-Substrat, die Testsubstanz Bromelain F9 sowie die Kunststoffküvetten werden auf 37°C in einem Inkubator vortemperiert. In die Meßküvette werden 20 ml der Plasma-probe, 500 ml der Streptokinase (gebrauchsfertige Test-Kit-Lösung), Urokinase (1U/ml), tPA=Actilyse® (0.58 x 10⁶ I.E./ml) oder der Bromelain F9 - Lösung pipettiert. Nach Vermischung wird die Lösung 5 min bei 37°C inkubiert. Die Reaktion wird durch Zugabe von 100 ml Plasmin-Substrat (gebrauchsfertige Test-Kitlsösug) gestartet. Die Extinktion bei 405 nm wird in Abhängigkeit von der Probenkonzentration und der Zeit vermessen.

**Tabelle 1**

| Fibrinolytische Aktivität von Streptokinase, Bromelain F9 und Bromelain Base Powder im Plasminogen-Test | | | | | |
|---|---|---|---|---|---|
| Zeit (s) | Streptokinase (Kit) | Bromelain F9 (µg/ml) | | | Bromelain Base Powder 50 µg/ml |
| | | 5 | 10 | 30 | |
| 30 | 0,284 | 0,23 | 0,315 | 0,304 | 0,356 |
| 60 | 0,523 | 0,424 | 0,485 | 0,559 | 0,449 |
| 120 | 0,741 | 0,610 | 0,611 | 0,795 | 0,507 |
| 180 | 1,078 | 0,929 | 0,929 | 1,036 | 0,551 |

Wie aus Tabelle 1 ersichtlich, zeigt Bromelain F9 im kinetischen Test eine der Streptokinase vergleichbare Wirkung. Die Wirkung von Bromelain F9 ist zeit- und konzentrationsabhängig, der Maximaleffekt wird bei 30 mg/ml (1.0 U/mg) erreicht. Bromelain F9 ist bereits bei einer Konzentration von 5mg/ml (E = 0.929) der Wirkung des Bromelain-Base Powder (0.4 U/mg) bei einer Konzentration von 50 mg/ml (E = 0.55) überlegen.

### Beispiel 2

Das Ziel dieses Experiments besteht darin, zu überprüfen, ob und inwieweit die Kombination von Bromelain F9 mit Streptokinase der Wirkung von Streptokinase alleine überlegen ist.

**Tabelle 2**

| Fibrinolytische Aktivität von Streptokinase alleine und in Kombination mit Bromelain F9 im Plasminogen-Test | | |
|---|---|---|
| Zeit (s) | Streptokinase (Kit) | Streptokinase + Bromelain F9 |
| 30 | 0,284 | 0,246 |
| 60 | 0,523 | 0,479 |
| 120 | 0,741 | 0,728 |
| 180 | 1,078 | 0,939 |

Wie aus Tabelle 2 ersichtlich, ist die Kombination von Bromelain F9 (10 mg/ml) mit Streptokinase der Wirkung von Streptokinase alleine im Plasminogentest nieht überlegen.

Dies kann dahingehend interpretiert werden, daß die Wirkung von Bromelain F9 auf die Fibrinolyse (Bildung von Plasmin) ähnlich ausgeprägt ist, wie die von Streptokinase, jedoch 10- fach (bezogen auf die chemische Konzentration) über der von Bromelain Base Powder liegt. Die Wirkung von Bromelain F9 ist konzentrations-und zeitabhängig. Die Kinetiken entsprechen denen von Streptokinase alleine in diesem System.

### Beispiel 3

In diesem Experiment wird ein Vergleich der fibrinolytischen Aktivitäten von Urokinase, Gewebsplasminogenaktivator (tPA) und deren Kombinationen mit Bromelain F9 durchgeführt.

**Tabelle 3**

| Fibrinolytische Aktivität von Urokinase, tPA alleine und der Kombinationen mit Bromelain F9 im Plasminogen-Test | | | | |
|---|---|---|---|---|
| Zeit (s) | Urokinase (1U/ml) | tPA 0,58x10⁶ I.E./ml | Urokinase + Bromelain F9 (10 µg/ml) | tPA + Bromelain F9 (10 µg/ml) |
| 30 | 0,2216 | 0,2315 | 0,2757 | 0,2417 |
| 60 | 0,3517 | 0,3215 | 0,3888 | 0,3124 |
| 120 | 0,5830 | 0,4469 | 0,5244 | 0,4680 |
| 180 | 0,7970 | 0,7899 | 0,6640 | 0,7553 |

Wie aus dem Vergleich der in Tabelle 1 und Tabelle 3 gezeigten Werte hervorgeht, bewirkt Streptokinase in diesem Testsystem im Vergleich zu Urokinase und tPA eine stärkere Plasminogen-Umsetzung. Die Wirkung von 30 mg/ml Bromelain F9 (Tabelle 1, 3) entspricht der Wirkung von Streptokinase und ist der Wirkung von Bromelain Base Powder überlegen. Bei Kombination von Bromelain F9 mit vorstehenden Plasminogen-Aktivatoren sind keine stärkeren Wirkungen im Vergleich zur alleinigen Wirkung von Urokinase und tPA, ebenso wie für Streptokinase (Tabelle 2) nachweisbar.

### Wirkung von Bromelain F9 auf die Fibrin-Bildung aus humanem Plasma von gesunden Spendern

Hierbei soll überprüft werden, ob und inwieweit Bromelain F9 die Thrombin-induzierte Fibrin-Bildung aus humanem Plasma beeinflußt.

### Beispiel 4

Ausgangsmaterial ist Citratplasma gesunder Spender, welches mit Bromelain F9 bei 37°C vorinkubiert und anschließend mit Thrombin versetzt wird. Pro Testansatz werden zu 0,05 ml Citratplasma 0,02 ml Proteaselösung pipettiert und für 1 Std inkubiert. Anschließend erfolgt die Zugabe von 0.01 ml Thrombin (0.2 U/ml) und eine Inkubation von 10 min im Wasserbad bei 37°C. Die Fibrinbildung wird semiquantitativ, organoleptisch unter dem Invertmikroskop (20-fache Vergrößerung) bewertet.

Dabei wird gefunden, daß Bromelain F9 (100 mg/ml) ebenso wie Streptokinase die Thrombin-induzierte Fibrinbildung aus Citratplasma vollständig verhindert. Auf der Basis der eingesetzten chemischen Konzentration ist Bromelain F9 um den Faktor 2 wirksamer als Bromelain Base Powder. Dagegen ist Papain (100 mg/ml, spezifische Aktivität 7,1 U/mg) unter diesen Bedingungen wirkungslos.

### Wirkung von Bromelain F9 auf die Adhäsion von humanen Thrombozyten an BKEz-7 Rinder-Endothelzellen

Aus humanem Vollblut isolierte Thrombozyten werden mit dem Fluoreszenzfarbstoff 2,7-Bis-(2-carboxyethyl)-5,6-carboxyfluoresceinacetoxymethylester markiert. Permanente BKEz-7 Rinderaorta-Zellen (11.-22. Passage) werden zu 60.000 Zellen pro Vertiefung in eine 96 Mikrotiterplatte pipettiert und über Nacht inkubiert. Für den Thrombozyten-Endothelzell-Adhäsions-Assay sind 5x10⁷ Thrombozyten nach einer Inkubationszeit von 15 min bei 37°C optimal. Die Entfernung der nichtgebundenen Thrombozyten erfolgt durch zweimaliges Waschen der Zellen mit KRB-Puffer (Krebs-Ringer-Bicarbonatpuffer mit 5,6 mMol Glucose + 1 % BSA).

### Beispiel 5

Im Versuchsansatz wird geprüft, welche Wirkung Bromelain F9 auf bereits adhärente Thrombozyten aufweist. Nach Durchführung des Thrombozyten-Endothelzell-Adhäsions-Assays werden die adhärenten Thrombozyten (stimuliert mit 0.2 U/ml Thrombin) mit Bromelain F9 (0.01 mg/ml) für 10 min bei 37°C inkubiert. Als Vergleich wird Bromelain Base Powder (0.1 mg/ml) mitgeführt. Die resultierenden Thrombozyten-Bindungen an die Endothelzellen werden mit denen der Proteaseunbehandelten Proben verglichen. Wie aus der Tabelle 4 ersichtlich, reduziert Bromelain F9 die Thrombozytenbindung um 32% (68% Bindung), während Bromelain Base Powder erst bei einer Konzentration von 0.1 mg/ml wirksam ist, mit einer Abnahme der Thrombozytenbindung um 40% (60% Bindung).

**Tabelle 4**

| Adhäsion von Thrombozyten an BKEz-7 Endothelzellen unter dem Einfluß von Bromelain F9 | | | |
|---|---|---|---|
| - Thrombin | + Thrombin (0.2 U/ml) | +Bromelain F9 (0.01 µg/ml) | +Bromelain Base Powder (0.1 µg/ml) |
| % Adhäsion | | | |
| 61* | 100 | 68* | 60* |

| | | | |
|---|---|---|---|
| Die gemessenen Fluoreszenzintensitäten der Thrombin-stimulierten, adhärierten Thrombozyten werden auf 100% normiert; * p<0.001 (t-Test); im Vergleich zu den adhärenten, Thrombin-stimulierten Thrombozyten sind diese Unterschiede statistisch signifikant. | | | |

### Beispiel 6

Isolierte humane Thrombozyten (5x10⁷/ml) werden mit Bromelain F9 und Bromelain Base Powder bei unterschiedlichen Konzentrationen 15 min bei Raumtemperatur inkubiert, die Proteasen werden durch Zentrifugation (1000 x g) und Waschen entfernt, die Thrombozyten in 1 ml KRB-Puffer (siehe vorstehend) resuspendiert, mit 0.2 U/ml Thrombin inkubiert und in dem Adhäsions-Assay an BKEz-7 Zellen eingesetzt. Die Resultate sind in Tabelle 5 dargestellt.

**Tabelle 5**

| Adhäsion von Thrombozyten an BKEz-7 Endothelzellen unter dem Einfluß von Bromelain F9 und Bromelain Base Powder | | | | |
|---|---|---|---|---|
| - Thrombin | + Thrombin (0,2 U/ml) | +Bromelain F9 (µg/ml) | | +Bromelain Base Powder 0,1 µg/ml |
| | | 0,005 | 0,01 | |
| | | % Adhäsion | | |
| 61* | 100 | 86* | 75* | 69* |

| | | | | |
|---|---|---|---|---|
| * p< 0.001 (t-Test); im Vergleich zu den adhärenten, Thrombin-stimulierten Thrombozyten sind diese Unterschiede statistisch signifikant. | | | | |

Wie aus der Tabelle 5 ersichtlich, zeigt Bromelain F9 eine konzentrationsabhängige Hemmung der Thrombozyten-Adhäsion an die Endothelzellen. Eine geringere Abnahme der Thrombozyten-Adhäsion wird für Bromelain Base Powder bei einer Konzentration von 0.1 mg/ml bestimmt.

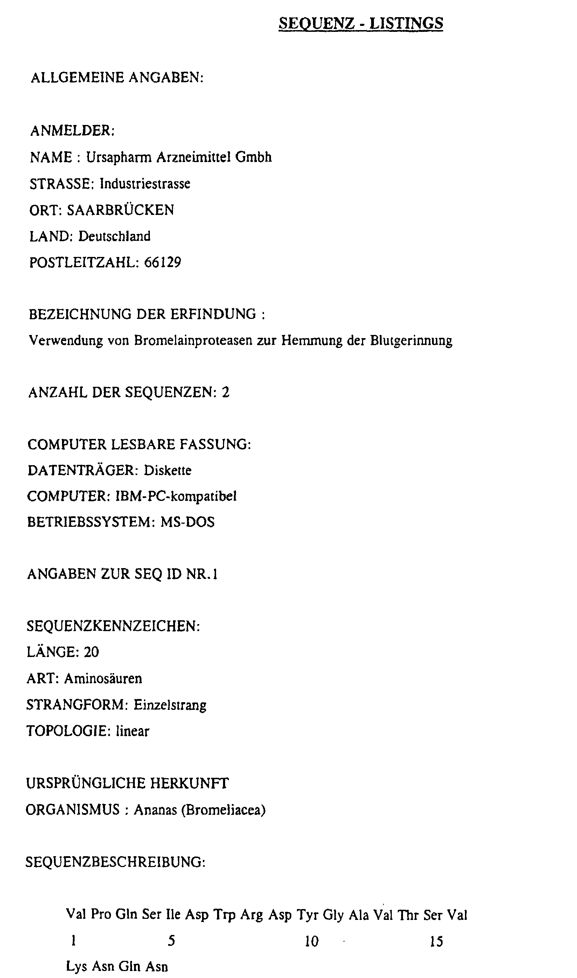

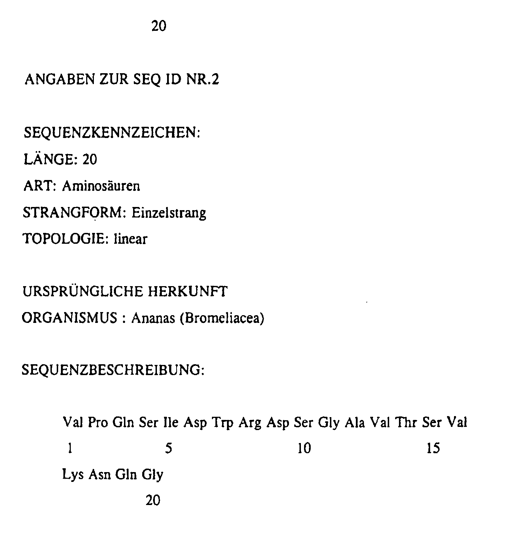

## Patentansprüche

1. Verwendung von Bromelainproteinasen zur Herstellung eines Medikamentes zur Hemmung der Blutgerinnung, wobei die Bromelainproteinasen ausgewählt sind aus der Gruppe bestehend aus:
a) einer basischen Bromelainprotease, die
- ein Molekulargewicht von etwa 24,4 KDa aufweist,
- ein Optimum der Aktivität bei einem pH-Wert im Bereich von 4 bis 5,5 besitzt, und
- die folgende Aminosäuresequenz umfaßt: 
Val Pro Gln Ser Ile Asp Trp Arg Asp Tyr
Gly Ala Val Thr Ser Val Lys Asn Gln Asn
 und/oder
b) einer basischen Bromelainprotease, die
- ein Molekulargewicht von etwa 24,5 KDa aufweist,
- ein Optimum der Aktivität bei einem pH-Wert im Bereich von 3,5 bis 5 besitzt, und
- die folgende Aminosäuresequenz umfaßt: 
Val Pro Gln Ser Ile Asp Trp Arg Asp Tyr
Gly Ala Val Thr Ser Val Lys Asn Gln Asn
und/oder
c) einer basischen Bromelainprotease, die
- ein Molekulargewicht von etwa 23,4 KDa aufweist,
- ein Optimum der Aktivität bei einem pH-Wert im Bereich von 6 bis 8 besitzt, und
- die folgende Aminosäuresequenz umfaßt:

Val Pro Gln Ser Ile Asp Trp Arg Asp Ser
Gly Ala Val Thr Ser Val Lys Asn Gln Gly

2. Verwendung nach Anspruch 1, wobei die Plasmin-bildung stimuliert wird.

3. Verwendung nach Anspruch 1, wobei die Fibrin-Bildung gehemmt wird.

4. Verwendung nach Anspruch 1, wobei die Adhäsion von Thrombozyten an Endothelzellen gehemmt wird.

## Claims

1. Use of bromelaine proteinases for the preparation of a medicament for inhibiting blood coagulation, the bromelaine proteinases being selected from the group consisting of:
a) a basic bromelaine protease having:
- a molecular weight of about 24.4 KDa,
- an optimal activity at a pH value in the range of 4 to 5.5, and
- comprising the following amino acid sequence: 
Val Pro Gln Ser Ile Asp Trp Arg Asp Tyr
Gly Ala Val Thr Ser Val Lys Asn Gln Asn
 and/or
b) a basic bromelaine protease having:
- a molecular weight of about 24.5 KDa,
- an optimal activity at a pH value in the range of 3.5 to 5, and
- comprising the following amino acid sequence: 
Val Pro Gln Ser Ile Asp Trp Arg Asp Tyr
Gly Ala Val Thr Ser Val Lys Asn Gln Asn
 and/or
c) a basic bromelaine protease having:
- a molecular weight of about 23.4 KDa,
- an optimal activity at a pH value in the range of 6 to 8, and
- comprising the following amino acid sequence:

Val Pro Gln Ser Ile Asp Trp Arg Asp Ser
Gly Ala Val Thr Ser Val Lys Asn Gln Gly

2. The use according to claim 1, wherein the plasmin production is stimulated.

3. The use according to claim 1, wherein the production of fibrin is inhibited.

4. The use according to claim 1, wherein the adhesion of thrombocytes to endothelium cells is inhibited.

## Revendications

1. Utilisation de bromélaïneprotéinases en vue de la production d'un médicament pour l'inhibition de la coagulation sanguine, dans le cadre de laquelle les bromélaïneprotéinases sont choisies dans le groupe constitué:
a) d'une bromélaïneprotéinase basique qui possède :
- un poids moléculaire d'environ 24,4 KDa,
- un optimum de son activité pour une valeur du pH dans le domaine de 4 à 5,5, et
- la séquence d'acides aminés suivante : 
Val Pro Gln Ser Ile Asp Trp Arg Asp Tyr
Gly Ala Val Thr Ser Val Lys Asn Gln Asn
et / ou
b) d'une bromélaïneprotéinase basique qui possède :
- un poids moléculaire d'environ 24,5 KDa,
- un optimum de son activité pour une valeur du pH dans le domaine de 3,5 à 5, et
- la séquence d'acides aminés suivante : 
Val Pro Gln Ser Ile Asp Trp Arg Asp Tyr
Gly Ala Val Thr Ser Val Lys Asn Gln Asn
et/ou
c) d'une bromélaïneprotéinase basique qui possède :
- un poids moléculaire d'environ 23,4 KDa,
- un optimum de son activité pour une valeur du pH dans le domaine de 6 à 8, et
- la séquence d'acides aminés suivante :

Val Pro Gln Ser Ile Asp Trp Arg Asp Ser
Gly Ala Val Thr Ser Val Lys Asn Gln Gly

2. Utilisation suivant la revendication 1, dans laquelle la formation de plasmine est stimulée.

3. Utilisation suivant la revendication 1, dans laquelle la formation de fibrine est inhibée.

4. Utilisation suivant la revendication 1, dans laquelle l'adhésion de thrombocytes à des cellules endothéliales est inhibée.
